# EUROPEAN PATENT SPECIFICATION

(11) **EP 1 491 160 B1**
(45) Date of publication and mention of the grant of the patent: **03.05.2006**
(21) Application number: 04014875.1
(22) Date of filing: 24.06.2004
(51) Int. Cl.: A61F 2/02, A61L 27/42

(54) **Ceramic member for medical implant and its production method**
Keramischer Körper für ein medizinisches Implantat und Herstellungsverfahren dafür
Elément céramique pour un implant médical et méthode pour sa production

(30) Priority: 25.06.2003 JP 2003181778
(43) Date of publication of application: 29.12.2004
(73) Proprietor: Kabushiki Kaisha Kobe Seiko Sho (Kobe Steel, Ltd.), Kobe-shi, Hyogo 651-8585 (JP)
(72) Inventor: Suzuki, Jun, K.K. Kobe Seiko Sho (Kobe Steel Ltd), Kobe-shi Hyogo 651-8585 (JP); Kudou, Takahiro K.K.Kobe Seiko Sho(Kobe Steel Ltd), Chuo-ku Kobe-shi Hyogo 651-8585 (JP); Matsushita, Tomiharu K.K. Kobe Seiko Sho, Chuo-ku Kobe-shi Hyogo 651-8585 (JP)
(74) Representative: TBK-Patent

(56) References cited:
- WO-A-02/087475
- US-A- 3 919 723
- US-A- 4 950 294
- US-B1- 6 270 347

## Description

### BACKGROUND OF THE INVENTION

### 1. Field of the Invention

This invention relates to a ceramic member for a medical implant which is adapted for use in artificial bone, artificial joint, and the like, to which the closest prior art is US-A-4950294. This invention also relates to a method for producing such a medical implant.

### 2. Description of Related Art

Zirconia, alumina, and their composite ceramic materials are often used as a material in producing an artificial bone, a bearing member of an artificial joint, or other implant members since they are non-toxic and they have excellent corrosion resistance, mechanical strength, and frictional properties. Since they have poor binding capability with the bone, a bone cement is used when the ceramic material is fixedly secured to the bone. This bone cement, however, is associated with the problem of heat generation in the course of its curing, and in some cases, drop in the blood pressure of the patient. After a prolonged use, the bone cement also experiences cracking and loosening.

In view of the situation as described above, various methods have been proposed to thereby avoid the use of the bone cement. One such method is formation in the body of an apatite (in particular, hydroxyapatite) between the bone and the implant member, and bonding of the bone and the implant member by utilizing the thus formed apatite.

Formation of apatite on the surface of a ceramic material such as zirconia or alumina, however, is difficult, and various improvements have also been sought to enable such formation. For example, Japanese Patent Application Laid-Open No. 2002-186663 discloses an article comprising a substrate of zirconia or the like and a coating of zirconia in its crystal phase containing Zr-OH group (Conventional Example 1). In this Conventional Example 1, formation of the apatite nucleus in the body is induced by the Zr-OH group, and growth of the apatite crystal is promoted since the coating comprises crystal phase.

The article of Conventional Example 1, however, suffered from the risk of the apatite being readily peeled off the implant member due to the weak bonding of the apatite layer to the coating while the formation of the apatite takes place as described above when it is immersed in the simulated human blood plasma (a solution having an inorganic ion composition similar to that of the human blood plasma).

In view of the situation as described above, the inventors of the present invention have made an investigation to prevent such peeling and found that bond strength between the ceramic surface and the bone can be improved by finely roughening the surface of the implant member. More specifically, surface of a zirconia composite ceramic material (material for the implant member) is polished with alumina slurry, and the article is immersed in hydrofluoric acid solution to further etch the surface. The surface of the ceramic material is thereby roughened (Conventional Example 2). The implant member (ceramic member) and the apatite member formed on the implant member are then firmly bonded by the anchor effect realized by surface micro-irregularities of the roughened surface. It is to be noted that this Conventional Example 2 has been published in "Proceedings for 24th Meeting of Japanese Society of Biomaterials on November 29 to 30, 2002", Japanese Society of Biomaterials, page 137.

However, the inventors of the present invention have found in the further investigation that, in the case of Conventional Example 2, the mechanical properties inherent to the ceramic material are lost to some extent.

### SUMMARY OF THE INVENTION

In view of the situation as described above, an object of the present invention is to improve the drawback as described above, and provide a ceramic member for a medical implant which exhibits improved binding with the bone without detracting from the excellent mechanical properties inherent to the ceramic material. Another obj ect of the present invention is to provide a method for producing such ceramic member for a medical implant.

The ceramic member for a medical implant according to the present invention is defined in claim 1 and has a surface layer including a surface roughened region which faces a bone.

The method of the present invention which is capable of producing such ceramic member for a medical implant is defined in claim 7 and comprises the steps of etching the a ceramic member with a strong acid solution (hereinafter sometimes referred to as the etching step), and subjecting the etched ceramic material to a heat treatment at 1000 to 1800°C (hereinafter sometimes referred to as the heat treatment step).

In the intensive study carried out by the inventors of the present invention, it has been found that the reason for the loss of the mechanical properties in the Conventional Example 2 was the phase change that took place in the zirconia crystal phase at the surface of the ceramic material during the polishing of the ceramic member with the alumina slurry, and this has resulted in the loss of the mechanical properties.

For example, if change from tetragonal zirconia to monoclinic zirconia took place by the application of some energy to the tetragonal zirconia, this phase change would be associated with the alteration in the volume of about 4. 6%. This volume alteration associated with the phase change causes local minute breakage to thereby reduce the mechanical strength.

In contrast, when the ceramic surface is roughened by the combination of the strong acid treatment and the heat treatment step as in the case of the production method of the present invention, the crystal composition of the surface layer will be equivalent (or substantially equivalent) with the crystal composition before the surface roughening treatment. For example, when composition of the zirconia crystal phase was calculated by using the intensity of the peaks obtained by X ray diffraction of a zirconia/alumina composite ceramic material produced by mixing zirconia with 30% by volume of alumina, the composition before the surface roughening step was about 95% by mass of tetragonal zirconia and about 5% by mass of monoclinic zirconia, whereas the composition after the treatment with an aqueous solution of hydrofluoric acid (strong acid treatment) and a heat treatment at 1300°C for 3 hours (heat treatment step) was about 93% by mass of tetragonal zirconia and about 7% by mass of monoclinic zirconia when the composition of the zirconia crystal phase was evaluated by the same procedure. In other words, the alteration in the composition of the crystal phase was as low as 2 points in % by mass. The inventors have found that the mechanical strength does not substantially decrease when the alteration in the composition of the crystal phase is 10 points or less in % by mass.

It is also to be noted that, in the method of the present invention, the surface roughening of the ceramic surface is accomplished by the step of the etching with the strong acid solution (strong acid treatment). The bond between the crystal grains of the ceramic material, however, is weakened if the ceramic material were subjected only to such strong acid treatment. The crystals recover their firm bonding when the ceramic material is subsequently subjected to the heat treatment (heat treatment step) as has been done in the present method since the crystals undergo diffusion bonding during the heat treatment.

In the ceramic member according to the present invention, the ceramic member is defined by the content of the crystal phase of interest as described above because such comparison of the crystal phase of interest enables estimation how the crystal phase composition varies over the entire ceramic member. It is also to be noted that, in the ceramic member of the present invention, the crystal phase of interest is compared not between the composition before the treatment and the composition after the treatment but between the composition of the surface layer and the composition of the deeper position since, and the ceramic member is defined by such difference. Such definition has been adopted in the present invention since the deeper part of the ceramic member retains the crystal composition before the treatment, and the crystal composition of such part can be used as a contrast instead of the composition of the ceramic member before the treatment. The term "a deeper position" is used herein as a position which is at least twice deeper than the depth of the recess in the surface irregularities as described below (the depth of the macro-recess in the surface). To avoid the risk of the influence by the surface roughening treatment, the composition of the surface layer is preferably compared with the deepest part of the ceramic member at a depth at least twice deeper than the deepest recess (1000 µm) in the surface irregularities, namely, the part at a depth of at least 2000 µm.

In other words, if the difference in the content in % by mass of the crystal phase of interest between the surface roughened surface layer and the deeper position is within 10 points, the phase change of the crystal phase can be regarded to have not occurred (to have not substantially occurred) at the surface of the ceramic member. In such a case, the mechanical strength has been maintained since no breakage due to the volume change upon phase change has occurred. In addition, firm bonding of the apatite layer is enabled when the apatite layer is formed on the surface since the surface has been roughened.

It should be noted that the ceramic member for a medical implant of the present invention is not limited to the one produced by the strong acid treatment and the heat treatment step as described above. The ceramic members having the surface roughened by other methods, and the ceramic members that have been produced with the roughened surface are also within the scope of the present invention.

In addition, the ceramic member for a medical implant of the present invention may preferably have the projections (hereinafter sometimes referred to as micro-projections) at a density of 1 to 2500/100 µm² on the irregular surface region of the surface layer when observed by using a scanning electron microscope at a magnification of 10,000x to 20,000x. As described above, while the apatite is firmly bonded to the ceramic member by the anchor effect of the roughened surface, such anchor effect is even more enhanced by the presence of the micro-projections of 1 to 2500/100 µm².

Before further explanation, the process of the apatite formation is explained. When an article adapted for the apatite formation is immersed in a simulated human blood plasma, the apatite formed generally takes the form of domes at first, and the domes then develop into the apatite layer. Most apatite domes have a diameter in the range of 0.5 to 20 µm, and the size of the micro-projection that can have the anchor effect for the apatite dome, namely, the size of the micro-projection that can capture the dome to enhance the bonding is at the level of several hundred nanometers. This corresponds to the micro-projection density of about 1 to 2500/100 µm². More preferably, the micro-projections are formed at a density of not less than 30/100 µm² and not more than 550/100 µm². When the micro-projection density is too low, the anchor effect for the apatite layer will be insufficient while an excessively high micro-projection density may also result in the insufficient anchor effect since such high density may result in the excessively small recess (hereinafter sometimes referred to as a micro-recess) in the roughened surface layer or excessively small micro-projection to detract from the anchor effect. More preferably, the projection (micro-projection) and the recess (micro-recess) are randomly and irregularly distributed in the roughened surface layer.

In the present invention, the roughened surface as described above are formed on the surface of irregularities formed in the region of the ceramic member that faces the bone, where the recesses of the surface irregularities (hereinafter sometimes referred to as macro-recesses) have a size in planer view of 50 to 1000 µm and a density of 10 to 500/cm² when observed by a stereoscopic microscope at a magnification of 10x to 15x. A stereoscopic microscope is a type of optical microscope which is generally used at a magnification of about 5x to 80x for observation of various specimens and samples in their steric images.

In other words, the surface of the ceramic member preferably has surface irregularities including the macro-recesses when the surface is macroscopically observed, and such presence of the macro-recesses in the region facing the bone enables growth of the bone into the macro-recesses, and this may realize firm bonding between the bone and the ceramic member for the medical implant. The macro-recess may be either an independent hole or a pore communicating with adjacent pores.

In the present invention, the ceramic member preferably comprises a zirconia composite ceramic material since this material has an excellent mechanical strength and this material is also highly adapted for surface roughening.

In addition, ceramic member for a medical implant according to the present invention is preferably the one having a substance which has affinity for the bone (hereinafter sometimes referred to as the substance having the bone affinity) deposited at least in the recess (micro-recess) of the roughened surface of the surface layer.

Preferable examples of such substance having the bone affinity include calcium salt compounds. When the substance having the bone affinity such as calcium salt compounds is deposited in the recesses of the roughened surface, formation of the apatite is facilitated to enable speedy bonding of the ceramic member with the bone.

Such ceramic member is preferably produced by conducing the strong acid treatment and the heat treatment step as described above, and then depositing the bioactive substance (hereinafter sometimes referred to as a deposition step).

In addition, ceramic member for a medical implant according to the present invention is preferably the one having a coating containing an apatite as its main component on its outermost surface, and more preferably, the apatite is hydroxycyanoapatite (Ca₅(PO₄)₃OH). The presence of such apatite on the outermost surface enables an enhanced bonding of the ceramic member with the bone.

Such ceramic member is preferably produced by forming an apatite-based coating on the ceramic member that had undergone the deposition step, and more specifically, by immersing the ceramic member that had undergone the deposition step in a simulated human blood plasma having the inorganic ion composition similar to that of the human blood plasma to thereby form an apatite on its outermost surface.

### BRIEF DESCRIPTION OF THE DRAWINGS

FIG. 1 is a scanning electron micrograph of the ceramic member for a medical implant of Example 1 according to the present invention. This photomicrograph is a view from above.
FIG. 2 is a scanning electron micrograph of the ceramic member for a medical implant of Example 1 according to the present invention. This photomicrograph is a horizontal view.
FIG. 3 is a scanning electron micrograph of the ceramic member for a medical implant of Example 2 according to the present invention.
FIG. 4 is a graph showing the results of the thin film X ray diffraction of the surface of the sample of Example 2.
FIG. 5 is a scanning electron micrograph of the comparative sample (Sample No. 5) of Example 3.
FIG. 6 is a scanning electron micrograph of the ceramic member for a medical implant of Example 4 according to the present invention.
FIG. 7 is a graph showing the results of compositional analysis of the zirconia crystal phase at the surface of the Sample Nos. 6 to 8 in Example 5.

### DETAILED DESCRIPTION OF THE PREFERRED EMBODIMENTS

First, the method for producing the ceramic member for a medical implant according to one embodiment of the present invention is schematically described.

A composite ceramic material containing zirconia and alumina was used for the starting material to produce a ceramic member of the desired shape and size having surface irregularities. This member is washed in acetone and pure water, and immersed in a strong acid solution to etch the surface of the member (strong acid treatment step). The member is then ultrasonically cleaned in pure water, dried, and heat treated at a temperature equal to or higher than the temperature of diffusion heat treatment. Calcium salt compounds were deposited on the surface, and immersed in a simulated human blood plasma having an inorganic ion composition similar to biological human blood plasmas for several days to thereby form a film containing apatite as its main component.

The ceramic member of this embodiment produced as described above has surface irregularities in the region that faces the bone, and the surface of such surface irregularities is further surface roughened to form the surface layer including numerous micro-recesses to receive a substance having affinity for the bone. A coating containing apatite as its main component is formed as the outer most layer. The difference in the content in % by mass of the crystal phase of interest in the ceramic crystal phases constituting the surface layer and the content in % by mass of the crystal phase of interest at a deeper position is within 10 points.

Next, the surface irregularity as described above is described. The surface irregularities may be formed by various methods including (i) bonding of ceramic beads of predetermined shape and size onto the compact ceramic member by heating, and (ii) mixing of a pore producing agent which disappears in the course of the subsequent sintering such as ceramic beads of predetermined shape and size with the starting powder material for the ceramic member, and compacting and sintering the material to thereby form a ceramic article having the surface irregularities at the location where the pore producing agent had been present.

The macro-recess formed by such method is preferably sized to a diameter of 50 to 1000 µm and a density of 10 to 500 per cm² when observed by a stereoscopic microscope. The new bone (the bone) will grow into the macro-recess to thereby establish an anchor effect that enables firm bonding of the bone to the ceramic member for the medical implant. When the density of the macro-recess is excessively low, such anchor effect by the growth of the new bone into the micro-recess can not be expected. When the density of the macro-recess is excessively high, the strength of the surface irregularity itself may become insufficient and the ceramic member may fall short of the strength required for a medical implant while the anchor effect by the growth of the new bone into the macro-recess may be sufficient. The density of the micro-recess is more preferably in the range of 50 /cm² to 200 /cm². The macro-recess may be formed either as an independent recess or as a pore communicating with other pores. In the latter case, two macro-recess which are in communication with each other are counted as two recesses.

Next, the material constituting the ceramic member is described. The ceramic member may comprise a ceramic material such as zirconia, alumina, titania, calcia, or magnesia, and preferably, a composite ceramic material which is an assembly of crystals each having different composition such as zirconia/alumina composite ceramic material as described above. In the case of a composite ceramic material comprising crystal grains of different compositions, behavior of the crystal grains during the dissolution in the etching solution (an acid, an alkaline solution, or the like) will be different depending on their composition. To be more specific, when such composite ceramic material is exposed to an etching solution, crystal grains with higher solubility to the etching solution will dissolve before the crystal grains with lower solubility, leaving the crystal grains with the lower solubility in the form of fine projections (micro-projections), and the roughened surface is readily produced by the etching. When the ceramic material comprises single composition as in the case of 3Y zirconia, formation of micro-irregularities is rather difficult since, while the surface is roughened by the etching with strong acid solution, the surface will be smoothened during the subsequent diffusion bonding by the heat treatment due to the weak bonding between the crystal grains.

The most preferable ceramic materials are ceramic materials containing zirconia as the main component having alumina added thereto, which may further include a minute amount of silica, titania, calcia, magnesia, or other oxide. Such zirconia composite ceramic materials are provided with the mechanical properties of the level that enables use of such material for the medical implant.

Next, the surface layer is described. As described above, the surface of the ceramic material is roughened by the etching of the surface by the strong acid treatment. Exemplary strong acid solutions that may be used in the strong acid treatment include hydrofluoric acid, hydrochloric acid, sulfuric acid, nitric acid, phosphoric acid, and mixtures thereof. Use of hydrofluoric acid is preferable when the composite ceramic material is the one containing zirconia as its main component having alumina added thereto. In this case, the treatment with hydrofluoric acid will leave projections (micro-projections) of alumina since zirconia is more susceptible to dissolution by the hydrofluoric acid solution compared to zirconia.

As described above, etching with the strong acid solution leaves roughened surface with the crystal grains being left in the form of micro-projections at the level of several hundred nanometers. By varying the concentration and temperature of the strong acid solution, the speed of etching in this treatment can be adjusted, thereby enabling control of density and the like of the micro-projections. In particular, increase in the temperature is effective for increasing the etching efficiency, and the aqueous solution of hydrofluoric acid is preferably used at about 40 to about 80°C.

The bonding between the crystals which had been weakened in the strong acid treatment recovers its original firm bonding in the subsequent heat treatment (at 1000 to 1800°C) through diffusion bonding between the crystals. The temperature used in the heat treatment is preferably varied depending on the type of the ceramic material, and the temperature used is preferably equal to or higher than the diffusion bonding temperature of the particular ceramic material. For example, when the ceramic material used is a composite ceramic material containing zirconia as its component having alumina added thereto, the heat treatment is preferably conducted at a temperature exceeding about 1150°C since the diffusion of zirconia rapidly proceeds at a temperature around 1150°C. When the temperature of heat treatment is too low, diffusion bonding between the crystals will not occur, and the etched crystal grains may become detached. When the temperature of heat treatment is too high, growth of the crystal grains may take place to detract from the strength of the ceramic matrix. Accordingly, the heat treatment is preferably conducted in the case of zirconia at a temperature in the range of 1150 to 1500°C, and more preferably at not lower than 1200°C and not higher than 1450°C.

As described above, a roughened surface comprising the crystal grains constituting the ceramic material is formed on the surface of the ceramic member by the strong acid treatment step and the subsequent heat treatment step. The size of the thus formed micro-projections depends on the size of the crystal grains constituting the ceramic material, the size of the micro-projections is preferably in the range of 0. 1 to 10 µm for reliably establishing the anchor effect to the overlying apatite layer since the diameter of the apatite dome is about 0. 5 to 20 µm and the thickness of the apatite layer is also 0. 5 to 20 µm. In other words, the micro-projections may preferably have a width and a length in the range of 0.1 to 10 µm. The interval between the micro-projections (namely, the size of the micro-recess) is also in the range of 0. 1 to 10 µm in view of the diameter and the thickness of the apatite dome. In consideration of such size of the micro-projections and micro-recesses, the density of the micro-projections is preferably 1 to 2500 per area of 10 µm x 10 µm, and more preferably not less than 50 to not more than 500 per area of 10 µm x 10 µm when observed by SEM at a magnification of 10, 000x to 20, 000x. The thickness of the surface layer is preferably in the range of 0.1 to 10 µm since only one layer of micro-projections is required.

Next, the step of depositing the substance having affinity for the bone such as calcium salt compounds is described. The type of the substance having the bone affinity and the method of its deposition are not limited. However, the substance having the bone affinity is preferably a calcium salt compounds or bioactive glass. Of the calcium salt compounds, the most preferred is calcium phosphate since the calcium phosphate is a main component of the bone. The deposition methods which may be used include (1) formation of a coating by heating and melting a bioactive glass which has a relatively low melting point, (2) formation of a coating by immersion in an aqueous solution of calcium salt compound such as calcium phosphate, calcium carbonate, calcium nitrate, calcium hydroxide, calcium chloride or the like for crystallization, and (3) plasma spraying of hydroxyapatite. When calcium phosphate compounds are to be deposited, the ceramic member may be immersed alternately in the aqueous solution containing a calcium ion and the aqueous solution containing a phosphate ion to deposit crystals comprising calcium phosphate compounds.

When the substance having the bone affinity is to be deposited by using an aqueous solution of calcium phosphate compounds or the like, deposition of the calcium phosphate compounds on the ceramic material is substantially impossible if the ceramic material immediately after cutting were brought in contact with the aqueous solution due to the poor wettability of the ceramic material which would hamper retention of the aqueous solution. In contrast, the ceramic member of the present invention has a roughened surface exhibiting good wettability, and the coating of the calcium phosphate compounds is readily formed since the aqueous solution is readily retained in the micro-recesses of the roughened surface.

Most preferably, the substance having the bone affinity is deposited on the ceramic member such that the substance uniformly covers the roughened surface (surface formed with the micro-recesses and the micro-projections) without completely filling the micro-recesses. Although the firm bonding of the apatite layer is realized to some extent by the roughened surface of the ceramic member, the ceramic material does not have the apatite forming ability and bonding with the bone is difficult if the substance having the bone affinity were not deposited. Formation of the apatite as well as firm bonding are enabled by the deposition of the affinity substance. It is also to be noted that the deposition of the affinity substance at least in the micro-recesses should realize anchor effect for the apatite layer to enhance the firm bonding between the ceramic member and the apatite layer.

Next, the apatite coating formed on the outermost surface is described. As described above, a coating containing the apatite as its main component is formed by immersing the ceramic member in the simulated human blood plasma having an inorganic ion composition similar to the human blood plasma. The apatite formed in the simulated human blood plasma has the composition and the structure which are similar to the apatite included in the bone, and therefore, a smooth bonding with the bone can be expected if such apatite coating were formed. It is to be noted that the coating formed as described above generally contains a minute amount of calcium carbonate or magnesium carbonate in addition to the apatite, and the content of the apatite is generally at least about 70%.

The apatite layer newly formed in the simulated human blood plasma is firmly bonded to the underlying ceramic substrate, and the apatite layer is not peeled by the peeling test wherein an adhesive tape is adhered to the surface of the apatite layer and the tape is subsequently peeled off the surface by pulling the tape upward at a right angle to the surface (JIS K5400-8.5).

As described above, the ceramic member of this embodiment has a surface wherein the surface of the region formed with the macro-projection and the macro-recesses has been roughened, and the substance having the bone affinity has been deposited on the roughened surface, and the apatite coating has been further formed on the outermost surface. As a consequence, it can be estimated that, when this ceramic member is implanted in the living body, the bone will directly bond to the surface of the ceramic member by the intervening apatite, and simultaneously, the bone will grow and intrude into the macro-recess of the surface irregularities. Such microscopic direct bonding and the macroscopic anchoring by the bone growth synergistically act to realize the firm bonding of the ceramic member with the bone. It is to be noted that use of the ceramic member for the bearing member of an artificial joint can be expected when the ceramic member comprises a surface layer including the surface irregularity of about 5 mm and the underlying compact substrate, and use of the ceramic member for the artificial bone or artificial bone filler can be expected when the entire ceramic member comprises has irregular structure, namely, when the entire ceramic member is porous.

Next, the ceramic member for a medical implant and its production method of the present invention are described in further detail by referring to the following Examples which by no means limit the scope of the present invention. It should be apparent to those skilled in the art that these Examples may be varied or modified without deviating from the scope of the invention and such variation and modification are within the technical scope of the present invention.

### EXAMPLES

### Example 1

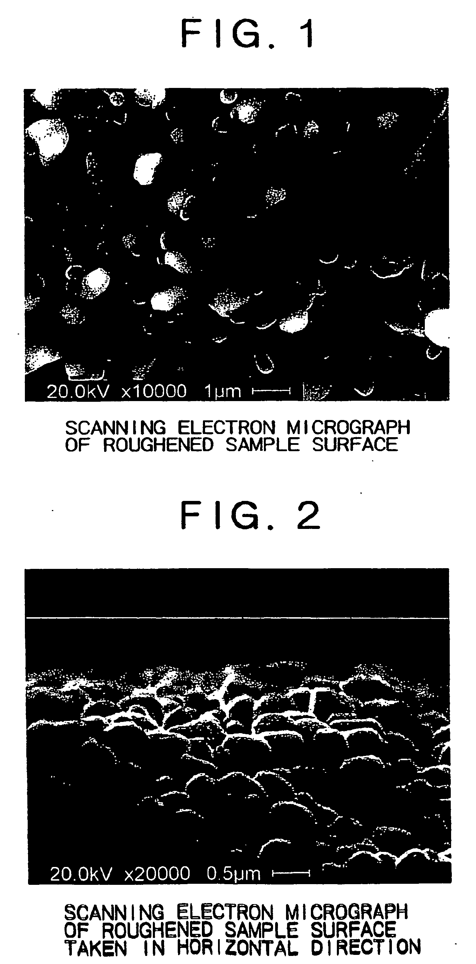

A starting powder material for zirconia/alumina composite ceramic material prepared by adding 30% by volume of alumina to zirconia was compacted and sintered, and worked into a piece of 10 mm x 10 mm x 3 mm. The resulting article was ultrasonically cleaned in acetone and pure water. This sample was immersed in a 12% by mass aqueous solution of hydrofluoric acid which had been heated to 60°C for 30 minutes (the strong acid treatment step), and after the recovery from the solution, the sample was ultrasonically cleaned in pure water for 10 minutes. After repeating the ultrasonic cleaning for 3 times, the sample was dried at room temperature, and then, heat treated at 1300°C for 3 hours (the heat treatment step) to cause diffusion bonding between the etched crystal grains. The roughened surface of the thus obtained sample was observed by using a scanning electron microscope (SEM). The scanning electron micrograph of the sample taken in vertical direction from the top is shown in FIG. 1. The scanning electron micrograph taken in horizontal direction is shown in FIG. 2.

As shown in FIG. 1, the roughened surface had the micro-projections with a width of about 0.3 to 1 µm at an interval of about 0. 5 to 2 µm. As shown in FIG. 2, the micro-projections had a height of about 0.3 to 1 µm, and the micro-projections were formed at a density of about 160/100 µm².

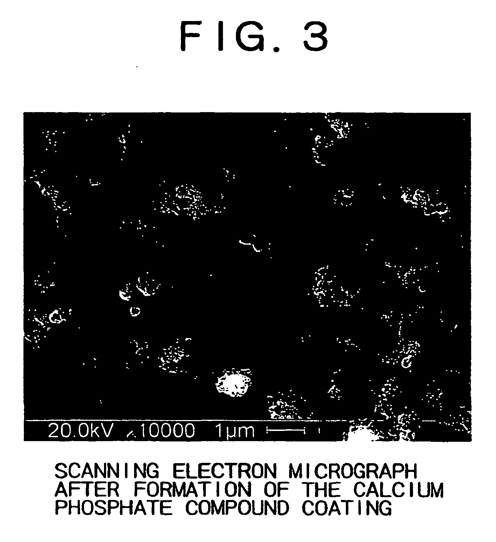

Next, calcium phosphate compounds were deposited on the surface of the sample prepared as described above. More specifically, the sample was immersed in 200mM aqueous solution of calcium chloride for 5 minutes, and after drying with no washing, the sample was immersed in 160mM aqueous solution of disodium hydrogenphosphate for 5 minutes (the deposition step). After repeating the immersion in the two types of solutions twice, the sample was washed with pure water to thereby form the film of calcium phosphate compounds. The sample was observed with SEM. The scanning electron micrograph is shown in FIG. 3.

As seen from FIG. 3, the calcium phosphate crystals are deposited without fully implanting the previously formed micro-proj ections. Analysis of the sample surface by thin film X ray diffraction confirmed the presence of weak peaks corresponding to the apatite and CaHPO₄(OH)·2H₂O.

The sample was evaluated by four point bending test for its flexural strength by the test procedure described in JIS R1601. The sample had a flexural strength comparable to the zirconia/alumina composite ceramics member which had not undergone the surface roughening treatments (the steps of strong acid treatment and the heat treatment). The sample was also measured by X ray diffraction for the content in % by mass of the monoclinic zirconia in the surface layer and at a deeper position. The difference in the monoclinic zirconia content was about 2 to 3 points.

### Example 2

The ceramics samples produced by repeating the procedure of Example 1 (the samples coated with the film of calcium phosphate compounds) were immersed in a simulated human blood plasma having an inorganic ion composition comparable to human blood plasmas at 37°C for 1 to 3 days to thereby form an apatite coating on the surface.

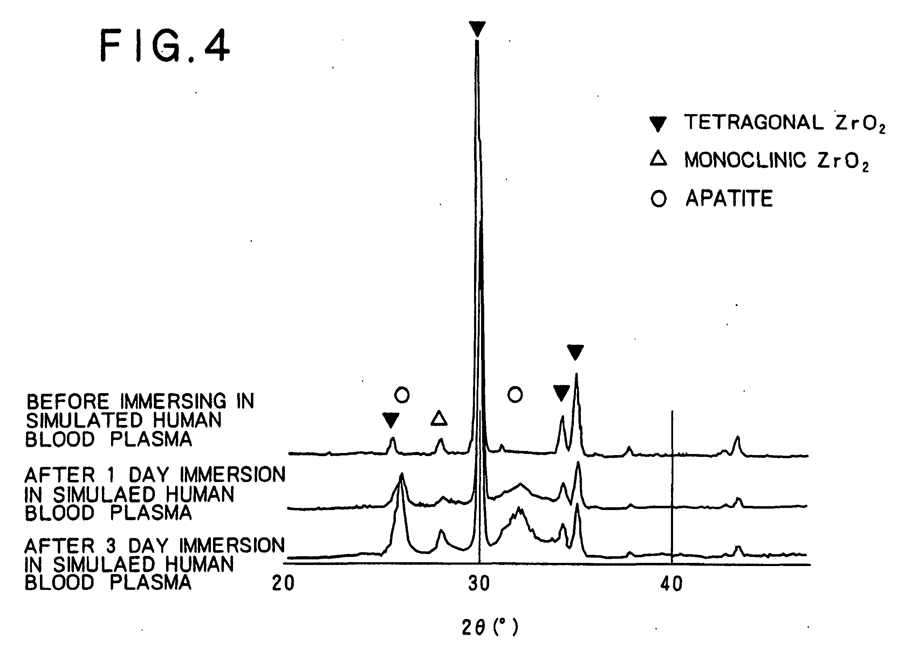

Analysis of the sample surface by thin film X ray diffraction confirmed the presence of peaks corresponding to the apatite as shown in FIG. 4 (the graph showing the results of thin film X ray diffraction). In FIG. 4, the peaks corresponding to the apatite are indicated by a blank circle, the peaks corresponding to the monoclinic zirconia are indicated by a blank triangle, and the peaks corresponding to the tetragonal zirconia are indicated by a black triangle.

The bond strength of between the apatite layer and the ceramic substrate was evaluated by tape peeling test according to the procedure defined in JIS K5400-8. 5. The apatite layer remained on the ceramic substrate and no peeling of the apatite layer was observed. This result indicates the firm bonding between the apatite layer and the ceramic substrate.

In this sample, the difference in the content in % by mass of the monoclinic zirconia between the surface layer and the deeper position was about 2 to 3 points

### Example 3

A starting powder material for zirconia/alumina composite ceramic material prepared by adding 30% by volume of alumina to zirconia was compacted and sintered, and worked into a piece of 10 mm x 10 mm x 3 mm. The resulting article was ultrasonically cleaned in acetone and pure water. This sample was then immersed in an aqueous solution of hydrofluoric acid for surface roughening (the strong acid treatment step). In this step of treating with the strong acid, the sample was treated with the aqueous solution of hydrofluoric acid under various conditions by varying the concentration, the temperature, and the time of immersion in the hydrofluoric acid as shown in Table 1 to thereby produce samples having the micro-projections of various densities. The samples having the micro-projections at a density of 25/100 µm² and 85/100 µm² were produced by using a 6% aqueous solution of hydrofluoric acid and adjusting the time and temperature of immersion (Sample Nos. 1 and 2), and the samples having the micro-projections at a density of 160/100 µm² and 570/100 µm² were produced by using a 12% aqueous solution of hydrofluoric acid and adjusting the time and temperature of immersion. A comparative sample with no surface roughening (Sample No. 5) was also produced by omitting the treatment with the aqueous solution of hydrofluoric acid.

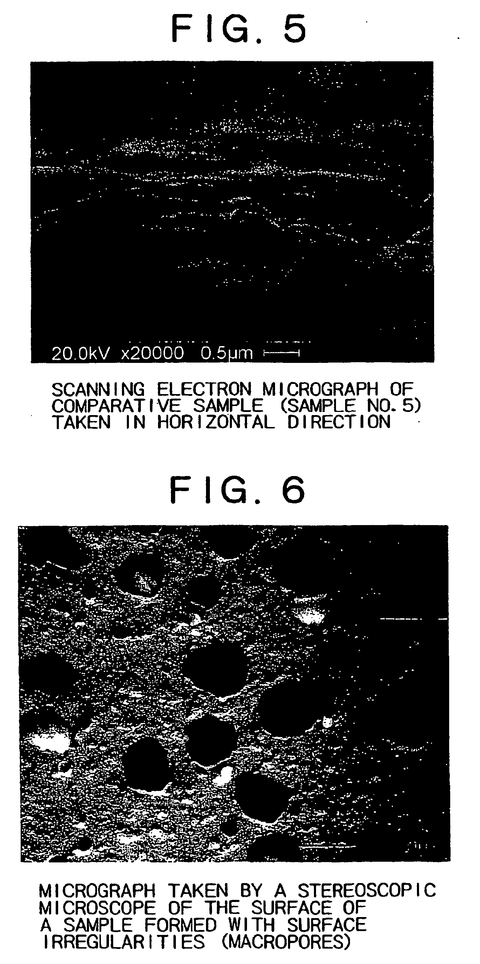

These samples were ultrasonically cleaned, dried, and heat treated (the heat treatment step), and covered with a coating of calcium phosphate compounds as in the case of Example 1. The samples were then immersed in a simulated human blood plasma for 3 days to form the apatite coating. The apatite coating was evaluated for its bond strength by the tape peeling test as described above. The result of the tape peeling test and the conditions of the apatite coating are shown in Table 1. The scanning electron micrograph of the surface of the comparative member (Sample No. 5) is shown in FIG. 5. It is to be noted that Sample No. 3 is the same as the one produced in Example 2.

**Table 1**

| Sample No. | Number of micro-projections (/100 µm²) | Formation of apatite layer | Result of peeling test |
|---|---|---|---|
| 1 | 25 | Yes | B |
| 2 | 85 | Yes | A |
| 3 | 160 | Yes | A |
| 4 | 570 | Yes | B |
| 5 | 0 | No | - |

### Formation of apatite layer

Yes: formation of the apatite layer was observed.

No: no formation of the apatite layer was observed.

### Result of the peeling test

A: no peeling
B: some peeling
C: complete peeling

As evident from Table 1, no apatite layer was formed on the comparative sample having no surface formed with surface irregularities. It is also evident that bond strength of the apatite layer to the ceramic member is rather insufficient in the case of Sample No. 1 formed with the micro-projections at a density of as low as 25/100 µm² or in the case of Sample No. 4 formed with the micro-projections at a density of as high as 570/100 µm². In contrast, Sample Nos. 2 and 3 formed with the micro-projections at a density of 85/100 µm² and 160/100 µm² exhibit excellent bond strength with the apatite layer. It is to be noted that, when the Sample Nos. 1 to 4 were measured by X ray diffraction for the content in % by mass of the monoclinic zirconia in the surface layer and at a deeper position, the difference in the monoclinic zirconia content was about 2 to 3 points. When the Sample Nos. 1 to 5 were evaluated by four point bending test for its flexural strength by the test procedure described in JIS R1601, no substantial difference was noted for the Sample Nos. 1 to 5.

### Example 4

A starting powder material for zirconia/alumina composite ceramic material prepared by adding 30% by volume of alumina to zirconia was mixed with organic beads (pore producing agent which disappears in the course of the subsequent sintering), and the mixture was compacted and sintered to produce a macroporous sample (a sample having surface irregularities) wherein pores with a diameter of about 200 µm and 800 µm (macro-recesses) have been formed. The picture taken by a stereoscopic microscope is shown in FIG. 6.

This sample has smaller pores with the diameter of about 200 µm and larger pores with the diameter of about 800 µm, and these smaller and larger pores (macro-recesses) were present at a density of about 200/cm² when observed with a stereoscopic microscope at a magnification of 10x.

Next, the sample was immersed in a 12% by mass aqueous solution of hydrofluoric acid which had been heated to 60°C for 30 minutes (the strong acid treatment step), and after the recovery from the solution, the sample was ultrasonically cleaned in pure water for 10 minutes. After repeating the ultrasonic cleaning for 3 times, the sample was dried at room temperature, and then, heat treated at 1300°C for 3 hours (the heat treatment step) to cause diffusion bonding between the etched crystal grains. The surface of the sample was observed with SEM to confirm that the surface had been uniformly roughened including the interior of the macro-recesses.

Next, this sample (the sample having surface irregularities which had been further surface roughened) was treated as described above for Example 1 to form a film of calcium phosphate compounds on its surface (the deposition step). The sample was then immersed in a simulated human blood plasma at 37°C for 1 to 3 days to thereby form an apatite coating on the surface.

The resulting sample had a substantially uniform apatite coating formed along the surface of the surface irregularities. When the sample was evaluated by four point bending test for its flexural strength by the test procedure described in JIS R1601, the sample exhibited a strength comparable with the sample of macroporous zirconia/alumina composite ceramics having the surface irregularities but which had not undergone the surface roughening treatment (the strong acid treatment step and the heat treatment step). When the sample was measured by X ray diffraction for the content in % by mass of the monoclinic zirconia in the surface layer and at a deeper position, the difference in the monoclinic zirconia content was about 2 to 3 points.

### Example 5

A starting powder material for zirconia/alumina composite ceramic material prepared by adding 30% by volume of alumina to zirconia was compacted and sintered, and worked into a piece of 10 mm x 10 mm x 3 mm. The resulting article was ultrasonically cleaned in acetone and pure water. Three samples were produced, and one sample was used with no further treatment (Sample No. 6: "non-treated sample"). The other one sample was polished with #600 alumina slurry, immersed in a 12% by mass aqueous solution of hydrofluoric acid at room temperature for 30 minutes, and ultrasonically cleaned in pure water for 10 minutes three times (Sample No. 7: "polished and etched sample"), and the last one sample was immersed in a 12% by mass aqueous solution of hydrofluoric acid at 60°C for 30 minutes (the strong acid treatment step), ultrasonically cleaned in pure water for 10 minutes three times, and heated at 1300°C for 3 hours (the heat treatment step) (Sample No. 8: "etched and heated samples").

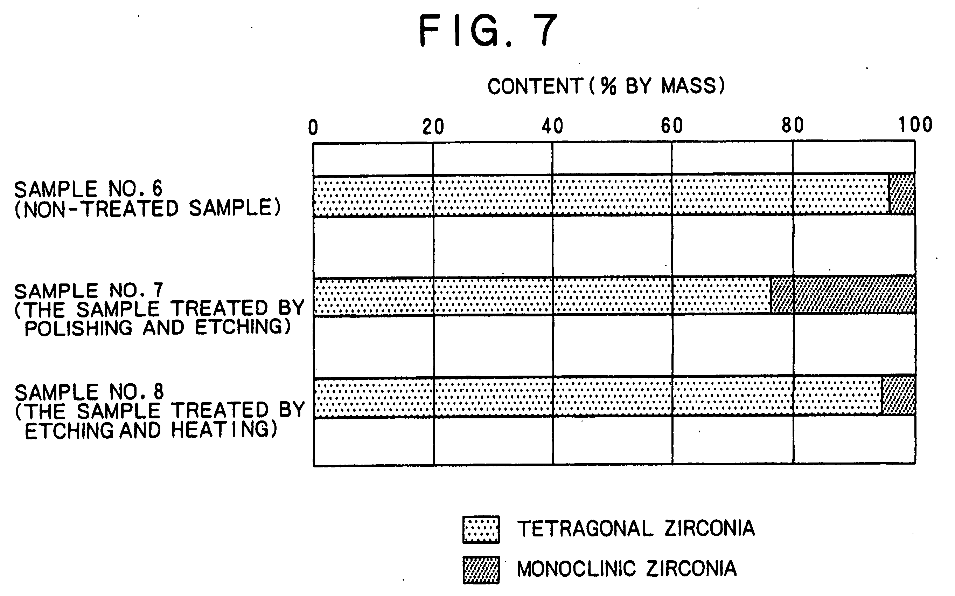

These Sample Nos. 6 to 8 were analyzed by X ray diffraction for the composition of the zirconia crystal phase on their surface, and ratio of the tetragonal zirconia to the monoclinic zirconia was calculated from their peak intensities. The results of the analysis are shown in FIG. 7.

As demonstrated in FIG. 7, Sample No. 8 which had been subjected to the strong acid treatment (etching) and the heat treatment according to the present invention had a composition of the crystal phase substantially comparable to that of the non-treated Sample No. 6. In contrast, phase change in zirconia had occurred in Sample No. 7 which had been subjected to the polishing and the strong acid treatment (etching) as in the case of Conventional Example 2.

When Sample Nos. 7 and 8 were evaluated by four point bending test for its flexural strength by the test procedure described in JIS R1601, Sample No. 7 had a strength inferior to that of Sample No. 8. This demonstrates that phase change as in the case of Sample No. 7 invites decrease in the strength.

As demonstrated by the results as described above, the surface roughening conducted by the method according to the present invention is less likely to induce the phase change in zirconia, and this method is also virtually free from the risk of inducing the loss of mechanical properties.

The ceramic member for a medical implant according to the present invention has the merit that the apatite layer formed on the surface is firmly bonded to the underlying ceramic member, and that it retains various properties inherent to the ceramic material including the excellent mechanical properties. The production method of the present invention is capable of forming a roughened surface which allows firm bonding of the overlying apatite layer to the ceramic member without detracting from the mechanical properties, and such production is accomplished by a simple procedure.

Disclosed herein is a ceramic member for a medical implant which exhibits improved binding with the bone without detracting from the excellent mechanical properties inherent to the ceramic material. The ceramic member for a medical implant has a surface layer having a roughened surface. When attention is paid to one type of crystal phase of the crystal phases of the ceramics constituting the surface layer, difference between the content in % by mass of this crystal phase of interest in the surface layer and the content in % by mass of this crystal phase of interest deep in the ceramic member is within 10%.

## Claims

1. A ceramic member for a medical implant,
said ceramic member having a surface layer including a surface roughened region, wherein
when attention is paid to the content in % by mass of one type of crystal phase based on all types of crystal phases of a ceramic material constituting the surface layer, difference between the content in % by mass of said one type of crystal phase in the surface layer and the content in % by mass of said one type of crystal phase at a deeper position in the ceramic member is within 10%,
wherein said deeper position is a position which is at least twice deeper than the depth of a macro-recess,
said ceramic member being **characterized in that**
said surface roughened region is obtained by etching and includes, and
said surface roughened region is formed on an underlying irregular surface including macro-recesses having a size of 50 to 1000 µm.

2. The ceramic member for a medical implant according to claim 1 wherein said underlying irregular surface includes macro-recesses at a density of 10 to 500/cm² when observed by a stereoscopic microscope at a magnification of 10x to 15x.

3. The ceramic member for a medical implant according to claim 1 or 2 wherein a substance which has affinity for the bone is deposited at least in micro-recesses of said surface roughened region.

4. The ceramic member for a medical implant according to any one of claims 1 to 3 wherein said ceramic member comprises a zirconia composite ceramic material.

5. The ceramic member for a medical implant according to any one of claims 1 to 4 wherein said substance which has affinity for the bone is a calcium salt compound.

6. The ceramic member for a medical implant according to any one of claims 1 to 5 wherein said ceramic member has a film containing an apatite as its main component formed on its outermost surface.

7. A method for producing the ceramic member for a medical implant of any one of claims 1 to 6 comprising the steps of
etching the ceramic member with a strong acid solution, and
subjecting the etched ceramic member to a heat treatment at 1000 to 1800°C, the method resulting in a surface roughened region including micro-projections having a size of 0.1 to 10 µm at a density of 1 to 2500/100 µm² when observed by a scanning electron microscope at a magnification of 10,000x to 20,000x.

8. The method for producing a ceramic member for a medical implant according to claim 7 further comprising the subsequent step of depositing a bioactive substance.

9. The method for producing a ceramic member for a medical implant according to claim 8 further comprising the subsequent step of forming a film containing an apatite as its main component.

## Patentansprüche

1. Keramisches Element für ein medizinisches Implantat,
welches keramische Element eine Oberflächenschicht, die einen Bereich mit aufgerauhter Oberfläche enthält, besitzt, wobei
hinsichtlich des Anteils in Gewichtsprozent eines Typs einer Kristallphase, basierend auf allen Typen der Kristallphasen eines keramischen Materials, das die Oberflächenschicht zusammensetzt, ein Unterschied zwischen dem Anteil in Gewichtsprozent des einen Typs der Kristallphase in der Oberflächenschicht und dem Anteil in Gewichtsprozent des einen Typs der Kristallphase bei einer tieferen Position im keramischen Element innerhalb von 10% ist,
wobei die tiefere Position eine Position ist, die mindestens doppelt so tief wie die Tiefe einer Makro-Vertiefung ist,
welches keramische Element **dadurch gekennzeichnet ist, dass**
der Bereich mit aufgerauhter Oberfläche durch Ätzen erhalten wird und Mikro-Vorsprünge mit einer Größe von 0,1 bis 10 µm bei einer Dichte von 1 bis 2500/100 µm², bei Betrachtung durch ein Rasterelektronenmikroskop bei einer Vergrößerung von 10 000x bis 20 000x, enthält, und
der Bereich mit aufgerauhter Oberfläche auf einer darunter liegenden unregelmäßigen Oberfläche, die Makro-Vertiefungen mit einer Größe von 50 bis 1000 µm besitzt, erzeugt ist.

2. Keramisches Element für ein medizinisches Implantat nach Anspruch 1, wobei die darunter liegende unregelmäßige Oberfläche Makro-Vertiefungen bei einer Dichte von 10 bis 500/cm² enthält, bei Betrachtung durch ein stereoskopisches Mikroskop bei einer Vergrößerung von 10x bis 15x.

3. Keramisches Element für ein medizinisches Implantat nach Anspruch 1 oder 2, wobei eine Substanz, die eine Affinität zum Knochen hat, mindestens in Mikro-Vertiefungen des Bereichs mit aufgerauhter Oberfläche abgeschieden ist.

4. Keramisches Element für ein medizinisches Implantat nach einem der Ansprüche 1 bis 3, wobei das keramische Element ein keramisches Zirconiumdioxid-Kompositmaterial umfasst.

5. Keramisches Element für ein medizinisches Implantat nach einem der Ansprüche 1 bis 4, wobei die Substanz, die eine Affinität zum Knochen hat, eine Calciumsalz-Verbindung ist.

6. Keramisches Element für ein medizinisches Implantat nach einem der Ansprüche 1 bis 5, wobei das keramische Element einen auf dessen äußerster Oberfläche erzeugten Film hat, der als Hauptbestandteil einen Apatit enthält.

7. Verfahren zum Herstellen des keramischen Elements für ein medizinisches Implantat nach einem der Ansprüche 1 bis 6, umfassend die Schritte des
Ätzens des keramischen Elements mit einer starken Säurelösung, und
Unterziehens des geätzten keramischen Elements einer Wärmebehandlung bei 1000 bis 1800°C, wobei
das Verfahren resultiert in einem Bereich mit aufgerauhter Oberfläche, der Mikro-Vorsprünge mit einer Größe von 0,1 bis 10 µm bei einer Dichte von 1 bis 2500/100 µm², bei Betrachtung durch ein Rasterelektronenmikroskop bei einer Vergrößerung von 10000x bis 20000x, enthält.

8. Verfahren zum Herstellen eines keramischen Elements für ein medizinisches Implantat nach Anspruch 7, weiter umfassend den nachfolgenden Schritt des Abscheidens einer bioaktiven Substanz.

9. Verfahren zum Herstellen eines keramischen Elements für ein medizinisches Implantat nach Anspruch 8, weiter umfassend den nachfolgenden Schritt des Erzeugens eines Films, der einen Apatit als dessen Hauptbestandteil enthält.

## Revendications

1. Elément de céramique pour un implant médical,
ledit élément de céramique ayant une couche de surface incluant une région de surface rugueuse, dans laquelle,
lorsqu'on prête attention à la teneur en % en poids d'un type de phase cristalline basé sur tous les types de phases cristallines d'un matériau céramique constituant la couche de surface, la différence entre la teneur en % en poids dudit type de phase cristalline dans la couche de surface et la teneur en % en poids dudit type de phase cristalline à une position plus profonde dans l'élément de céramique se situe à l'intérieur d'une limite de 10 %,
dans lequel ladite position plus profonde est une position qui est au moins deux fois plus profonde que la profondeur d'un macro-renfoncement,
ledit élément de céramique étant **caractérisé en ce que** ladite région de surface rugueuse est obtenue par mordançage et inclut des micro-saillies ayant une taille de 0,1 à 10 µm à une densité de 1 à 2 500/100 µm² lorsque observé par un microscope électronique à balayage à un grossissement de x10 000 à x20 000, et
ladite région de surface rugueuse est formée sur une surface irrégulière sous-jacente incluant des macro-renfoncements ayant une taille de 50 à 1 000 µm.

2. Elément de céramique pour un implant médical selon la revendication 1, dans lequel ladite surface irrégulière sous-jacente inclut des macro-renfoncements à une densité de 10 à 500/cm² lorsque observée par un microscope stéréoscopique à un grossissement de x10 à x15.

3. Elément de céramique pour un implant médical selon la revendication 1 ou 2, dans lequel une substance qui possède une affinité pour l'os est déposée au moins dans les micro-renfoncements de ladite région de surface rugueuse.

4. Elément de céramique pour un implant médical selon n'importe laquelle des revendications 1 à 3, dans lequel ledit élément de céramique comprend un matériau céramique composite de zircone.

5. Elément de céramique pour un implant médical selon n'importe laquelle des revendications 1 à 4, dans lequel ladite substance qui possède une affinité pour l'os est un composé de sel de calcium.

6. Elément de céramique pour un implant médical selon n'importe laquelle des revendications 1 à 5, dans lequel ledit élément de céramique présente un film contenant une apatite comme composant principal formé sur sa surface externe.

7. Procédé de production de l'élément de céramique pour un implant médical selon n'importe laquelle des revendications 1 à 6, comprenant les étapes de mordançage de l'élément de céramique avec une solution d'acide fort, et
L'exposition de l'élément de céramique gravé à un traitement thermique à 1 000 à 1 800°C, le procédé aboutissant à une région de surface rugueuse incluant des micro-saillies ayant une taille de 0,1 à 10 µm à une densité de 1 à 2 500/100 µm² lorsque observée par un microscope électronique à balayage à un grossissement de x10 000 à x20 000.

8. Procédé de production d'un élément de céramique pour un implant médical selon la revendication 7, comprenant en outre l'étape ultérieure de dépôt d'une substance bioactive.

9. Procédé de production d'un élément de céramique pour un implant médical selon la revendication 8, comprenant en outre l'étape ultérieure de formation d'un film contenant une apatite comme composant principal.
